(19) Europäisches Patentamt
European Patent Office
Office européen des brevets

(11) EP 2 340 763 A1

(12) EUROPEAN PATENT APPLICATION

(43) Date of publication:
06.07.2011 Bulletin 2011/27

(51) Int Cl.:
A61B 5/00 (2006.01)          A61B 5/103 (2006.01)

(21) Application number: 11154239.5

(22) Date of filing: 20.09.2007

(84) Designated Contracting States:
AT BE BG CH CY CZ DE DK EE ES FI FR GB GR HU IE IS IT LI LT LU LV MC MT NL PL PT RO SE SI SK TR

(62) Document number(s) of the earlier application(s) in accordance with Art. 76 EPC:
07253721.0 / 2 039 287

(71) Applicant: Biocompatibles UK Limited
Farnham
Surrey GU9 8QL (GB)

(72) Inventors:
• Cotton, Symon
Great Gransden, SG19 3QS (GB)

• Morse, Robert
Cambridge, CB1 3PU (GB)
• Chellingworth, Mark
Vale of Glamorgan Wales, CF64 2NP (GB)

(74) Representative: Dawson, Elizabeth Ann et al
9-10 Savile Row
London W1S 3PF (GB)

Remarks:
This application was filed on 11-02-2011 as a divisional application to the application mentioned under INID code 62.

(54) **Method and apparatus for measuring collagen thickness**

(57) A camera (1) is provided which is operable to obtain a RGB and infra-red image of an area of illuminated skin (2). The obtained image is then passed to a computer (8) which determines the manner in which light returned by points on the surface of the illuminated area of skin (2) appearing in the obtained image varies due to variations in lighting intensity and surface geometry. The infra-red channel of the obtained image is then normalized on the basis of the determined variations and a measurement of collagen thickness can then be determined utilizing the processed infra-red channel of the obtained image. The determination of variations in intensities of light returned by points on the surface of the illuminated area of skin (2) can be achieved by processing an obtained image to generate a 3-D model of the surface being imaged and using the 3-D model to select and process pre-stored lighting level data. Alternatively variations in intensities of light can be determined by processing an obtained image to determine measurements of the concentrations of chromophores in the skin. A derived image of the expected appearance of the skin can then be generated and the manner in which light intensities are non-uniform can then be determined by comparing this derived image with the original obtained image.

Fig.2.

EP 2 340 763 A1

**Description**

The present application concerns methods and apparatus for measuring skin collagen thickness. More specifically, the present application concerns methods and apparatus for measuring skin collagen thickness which are non-invasive and which are suitable for measuring collagen thickness over areas of skin, such as the face which do not have a flat surface geometry.

The original research undertaken at the University of Birmingham argued that the Kubelka-Munk theory is sufficient to model light transport within skin. If exact scattering and absorption coefficients can be specified, then the Kubelka-Munk theory can be applied at each wavelength in the visible range and a corresponding remittance spectrum obtained. This predicted spectrum, which will determine the colour of the skin, will be dependent on the histological characteristics of the tissue. Three parameters capture most of the variation in remitted spectra from healthy skin. These three parameters are concentration of epidermal melanin, concentration of blood and thickness of the papillary dermal layer (collagen thickness).

Using the RGB response curves for a digital camera together with a model of the scattering and absorption characteristics of the skin, it is possible to calculate the set of image values which would be measured by a digital camera when skin with a known remittance spectrum $S(\lambda)$ is illuminated with light of known spectral characteristics $I(\lambda)$. This is done by calculating the convolution integral for each channel, given as,

$$i_{red} = \int I(\lambda)S(\lambda)R(\lambda)d\lambda \,,\; i_{green} = \int I(\lambda)S(\lambda)G(\lambda)d\lambda \,,\; i_{blue} = \int I(\lambda)S(\lambda)B(\lambda)d\lambda$$

where $R(\lambda)$, $G(\lambda)$ and $B(\lambda)$ are the response curves for the red, green and blue channels and $i_{red}$, $i_{blue}$ and $i_{green}$ are the corresponding values recorded by the camera at a given pixel.

By ranging through all potential combinations of melanin and blood concentrations and collagen thickness, it is possible to generate all possible spectra and therefore all possible sets of image values which could be measured by a digital camera. Once this information has been obtained a link can be established between image values and histological parameter values. This link can be expressed as a mathematical function. An image, acquired using a digital camera, consists of a large number of small pixels, each of which have a set of image values, ($i_{red}$, $i_{green}$ and $i_{blue}$). By applying the mathematical function, linking these image values to histological parameter values, it is possible to obtain values for melanin and blood concentration and collagen thickness at every pixel in an image of skin. This information can then be displayed in the form of histological parametric map.

Determining measurements of melanin concentration, blood concentration and collagen thickness directly from measurements of remitted light $S(\lambda)$ requires that an area of skin is illuminated with light of known spectral characteristics $I(\lambda)$. Using such an approach it is therefore necessary to follow a strict calibration procedure where lighting levels are strictly controlled. This limits the use of such an approach to analyzing small areas of skin as once larger areas of tissue are imaged, over which the surface geometry of the imaged tissue varies, calibration is no longer possible and analysis becomes inaccurate. Due to the required calibration procedures, it is typically only possible to produce a map over a small area of skin, currently 15mm diameter.

In order to overcome the problems arising due to strict calibration requirements an alternative technique has been developed. This is described in detail in Astron Clinica's prior patent application WO 04/010862. The technique relies on a mathematical function linking histological parameters with ratios of image values, rather than the actual image values. Determining measurements from ratios of image values removes the need for calibration. This can be demonstrated mathematically by considering the case where illumination which can be described by

$$I(\lambda) = \alpha_1 \overline{I}(\lambda) \,,$$

where $\alpha_1$ is a wavelength independent scaling factor which captures changes in illumination intensity and $\overline{I}(\lambda)$ captures the wavelength dependence of the incident light. The amount of light remitted from a tissue will depend on both the histological characteristics of the tissue and the angle of the tissue to the camera. The remitted spectrum can therefore be expressed as

$$S(\lambda) = \alpha_2 \overline{S}(\lambda)$$

where $\alpha_2$ is a wavelength independent scale factor which depends on the angle of the tissue to the camera and $S(\lambda)$ is the remitted spectrum which depends on the histology of the imaged tissue. Ratios of image values are now given as,

$$r_{greenOver\mathrm{Re}d} = \frac{\alpha \int \overline{I}(\lambda)S(\lambda)G(\lambda)d\lambda}{\alpha \int \overline{I}(\lambda)S(\lambda)R(\lambda)d\lambda} , \; r_{blueOver\mathrm{Re}d} = \frac{\alpha \int \overline{I}(\lambda)S(\lambda)B(\lambda)d\lambda}{\alpha \int \overline{I}(\lambda)S(\lambda)R(\lambda)d\lambda}.$$

where $\alpha=\alpha_1\alpha_2$. The factor $\alpha$, which captures all variation due to illumination changes and changes in surface geometry of the imaged tissue, will cancel out in each of the equations above leaving only wavelength dependent terms. Thus the image ratios can be seen to be independent of both illumination and surface geometry.

Variation in skin histology can then thought of in terms of a parameter space and spectra are computed, using the Kubelka-Munk model, which correspond to each point with parameter space. By applying the above equations it is then possible to calculate the two image ratios $r_{greenOverRed}$ and $r_{blueOverRed}$ which correspond to a given spectra. Using the above technique, measurements of blood and melanin concentrations can be made without having to control for surface geometry and lighting conditions.

Although effective, the described technique in WO 04/010862 is, however, limited to obtaining measurements of melanin and blood concentrations. The technique is not suitable for obtaining measurements of collagen as changes in collagen have an equal effect at every wavelength and therefore no effect on a ratio of two spectral measures.

An alternative system which is able to obtain a measurement of skin collagen thickness over wider areas of skin or areas of skin such as the face which do not have a flat surface geometry is therefore desirable.

In accordance with one aspect there is provided in the following an apparatus for measuring skin collagen thickness comprising:

  a camera operable to obtain an image of an area of illuminated skin;
  an illumination determination module comprising:

    a lighting calibration table storing data indicative of measurements of the manner in which lighting intensity varies in a volume where a sample of skin is to be imaged;
    a model generation module operable to process a received image to generate a 3 dimensional model of the surface of the area of skin being imaged; and
    a surface lighting determination module operable to process a 3 dimensional model generated by the model generation module and the data stored in the lighting calibration table to determine an estimate of the manner in which light returned by an area of skin being imaged varies due to variations in lighting intensity and surface geometry;

  an illumination normalisation module operable to process at least one colour channel of an obtained image on the basis of the determined variations; and
  a collagen determination module operable to determine a measurement of collagen thickness utilising a processed colour channel of an obtained image.

In accordance with another aspect of the present invention there is provided a method of measuring skin collagen thickness comprising:

  obtaining an image of an area of illuminated skin;
  storing measurements of the manner in which lighting intensity varies in a volume where a sample of skin is to be imaged;
  processing an obtained image to generate a 3 dimensional model of the surface of the area of skin being imaged;
  utilising the generated 3 dimensional model and the stored lighting measurements to determine an estimate of the manner in which light returned by an area of skin being imaged varies due to variations in lighting intensity and surface geometry;
  processing at least one colour channel of the obtained image on the basis of the determined variations; and
  determining a measurement of collagen thickness utilising the processed one colour channel of the obtained image.

In another aspect there is provided apparatus for measuring skin collagen thickness comprising:

a camera operable to obtain an image of an area of illuminated skin;

an illumination determination module operable to determine the manner in which light returned by points on the surface of the illuminated area of skin appearing in an obtained image varies due to variations in lighting intensity and surface geometry;

an illumination normalisation module operable to process at least one colour channel of an obtained image on the basis of the determined variations; and

a collagen determination module operable to determine a measurement of collagen thickness utilising a processed colour channel of an obtained image.

The illumination determination module comprises:

a lighting calibration table storing data indicative of measurements of the manner in which lighting intensity varies in a volume where a sample of skin is to be imaged;

a model generation module operable to process a received image to generate a 3 dimensional model of the surface of the area of skin(2) being imaged; and

a surface lighting determination module operable to process a 3 dimensional model generated by the model generation module and the data stored in the lighting calibration table to determine an estimate of the manner in which light returned by an area of skin being imaged varies due to variations in lighting intensity and surface geometry.

The apparatus may comprise a projector operable to project a pattern onto the surface of an area of skin being imaged, wherein said model generation module is operable to process a received image of an area of skin onto which a pattern is projected by said projector to generate a 3 dimensional model of the surface of the area of skin being imaged.

The surface lighting determination module is preferably operable to process a 3 dimensional model generated by the model generation module and the data stored in the lighting calibration table to determine an estimate of an expected intensity of light returned by an area of skin being imaged by:

determining an estimate of the intensity of light impinging on the surface of skin being imaged; and

determine an expected intensity of light returned by an area of skin by modifying the estimated intensity of light impinging on the surface of skin on the basis of the relative orientation of the surface of the skin relative to an image plane of a camera obtaining an image.

The collagen determination module preferably comprises a look up table associating measurements of a processed colour channel with thickness of collagen with which return said measurements of a processed colour channel.

The apparatus may further comprise:

a chromophore measurement module operable to process a received image to determine an estimate of the concentrations and distribution of blood and melanin in an area of skin appearing in an obtained image; wherein the collagen determination module is operable to determine a measurement of collagen thickness utilising estimates of the concentrations and distribution of blood and melanin in an area of generated by said chromophore measurement module and the processed colour channel of an obtained image.

The illumination determination module preferably comprises:

a chromophore measurement module operable to process a received image to determine an estimate of the concentrations and distribution of blood and melanin in an area of skin appearing in an obtained image; and

an lighting determination module operable to utilise a generated estimate of the concentrations and distribution of blood and melanin in an area of skin appearing in an obtained image to determine an estimate of the variations of the intensity of light returned by an illuminated area of skin which arise due to variations in lighting and surface geometry.

The collagen determination module preferably comprises a look up table associating measurements of a processed colour channel and concentrations of blood and melanin with thickness of collagen and concentrations of blood an melanin with which return said measurements of a processed colour channel.

The apparatus may further comprise a light source operable to illuminate an area of skin with polarised light; wherein said camera is operable to obtain an image of an area of skin illuminated by said light source via a polarising filter operable to filter light having the same polarization with which said light source is operable to illuminate said area of skin.

In another aspect there is provided a method of measuring skin collagen thickness comprising:

obtaining an image of an area of illuminated skin;
determining the manner in which light returned by points on the surface of the illuminated area of skin appearing in the obtained image varies due to variations in lighting intensity and surface geometry;
processing at least one colour channel of the obtained image on the basis of the determined variations; and
determining a measurement of collagen thickness utilising the processed one colour channel of the obtained image.

The determining the manner in which light returned by points on the surface of the illuminated area of skin appearing in the obtained image varies due to variations in lighting intensity and surface geometry preferably comprises:

storing measurements of the manner in which lighting intensity varies in a volume where a sample of skin is to be imaged;
processing an obtained image to generate a 3 dimensional model of the surface of the area of skin being imaged; and utilising the generated 3 dimensional model and the stored lighting measurements to determine an estimate of the manner in which light returned by an area of skin being imaged varies due to variations in lighting intensity and surface geometry.

Utilising a generated 3 dimensional model generated to determine an estimate of an expected intensity of light returned by an area of skin being imaged may comprise:

determining an estimate of the intensity of light impinging on the surface of skin being imaged; and determining an expected intensity of light returned by an area of skin by modifying the estimated intensity of light impinging on the surface of skin on the basis of the relative orientation of the surface of the skin relative to an image plane of a camera utilising the generated 3 dimensional model of the surface of the skin being imaged.

The method may further comprise:

processing a received image to determine an estimate of the concentrations and distribution of blood and melanin in an area of skin appearing in an obtained image; wherein determining a measurement of collagen thickness comprises determining a measurement of collagen thickness utilising the determined estimates of the concentrations and distribution of blood and melanin in an area of skin.

The step of determining expected intensities of light reflected by points on the surface of the illuminated area of skin appearing in the obtained image preferably comprises:

processing a received image to determine an estimate of the concentrations and distribution of blood and melanin in an area of appearing in an obtained image; and
utilising a generated estimate of the concentrations and distribution of blood and melanin in an area of skin appearing in an obtained image to determine an estimate of the variations of the intensity of light returned by an illuminated area of skin which arise due to variations in surface geometry.

There is also provided in the following a computer readable medium comprising computer interpretable instructions which when executed by a programmable computer cause the computer to perform any of the methods described above.

Further aspects and embodiments of the present invention will become apparent with reference to the accompanying drawings in which:

Figure 1 is a schematic cross sectional view through a layer of skin illustrating the structure of the skin and the interaction of that structure with incident light;

Figure 2 is a schematic block diagram of a first collagen thickness measurement system;

Figure 3 is a flow diagram of the processing performed by the collagen thickness measurement system of Figure 1;

Figure 4 is a schematic block diagram of a second collagen thickness measurement system;

Figure 5 is a flow diagram of the processing performed by the collagen thickness measurement system of Figure 4;

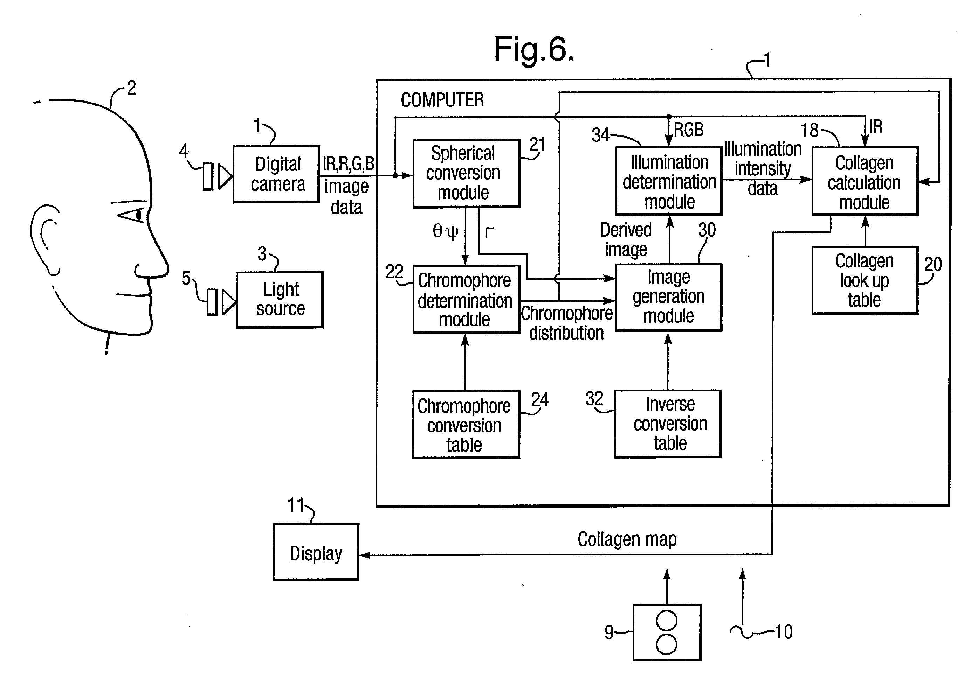

Figure 6 is a schematic block diagram of a third collagen thickness measurement system; and

Figure 7 is a flow diagram of the processing performed by collagen thickness measurement system of Figure 6.

Interaction of Light with the Skin

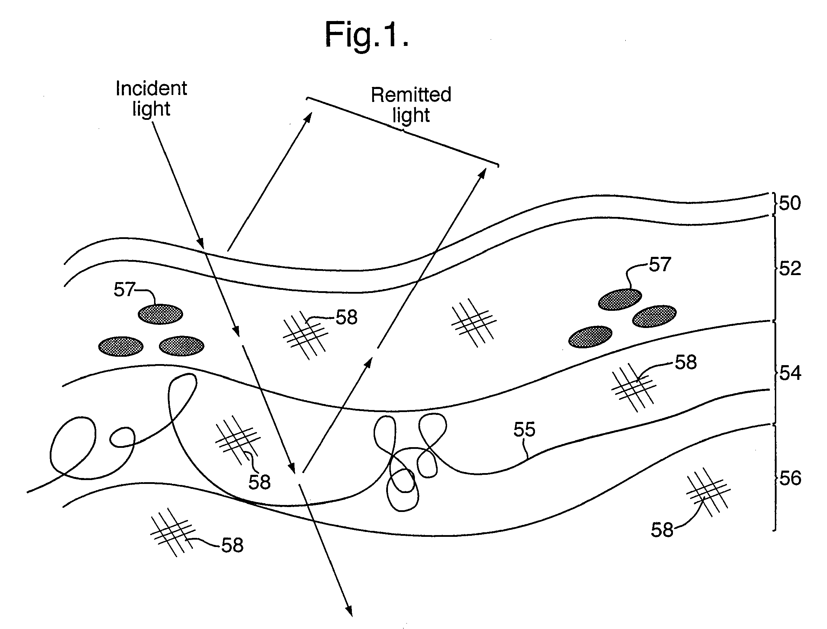

In order to aid understanding, prior to describing a number of specific embodiments of the present invention, the physical structure of skin and the interaction of skin with light will first be briefly explained with reference to Figure 1.

As shown in Figure 1, skin has a layered structure comprising an outer cornified layer 50, the epidermis 52, and the dermis which itself can be divided into the papillary dermis 54 which contains the blood supply 55 for the skin and the reticular dermis 56.

When light is incident on the skin, much of the light is immediately reflected when coming into contact with the outer cornified layer 50. A proportion of incident light does, however, pass through the cornified layer 50 and proceeds to interact with the constituents of the epidermis 52 and the papillary dermis 54.

As light passes through the epidermis 52 and the papillary dermis 54 the light is absorbed by various chromophores present in the skin, most notably chromophores such as haemoglobin present in the blood in blood vessels 55 in the papillary dermis 54, melanin, a pigment produced by melanocytes 57 in the epidermis 52 and collagen 58 a fibrous material present throughout the skin. By the time the incident light reaches the reticular dermis 56 the scattering of light is highly forward and therefore for that reason the reticular dermis 56 can for all intents and purposes be considered returning no light.

In addition to chromophores present in the epidermis 52 and papillary dermis 54 absorbing various wavelengths, certain structures in the skin most notably collagen 58 cause incident light to be reflected. The outward appearance of the skin can therefore be considered to be a mixture of the light immediately reflected by the cornified layer 50 and the remitted light which has interacted with the chromophores present in the epidermis 52 and the papillary dermis 54.

First System

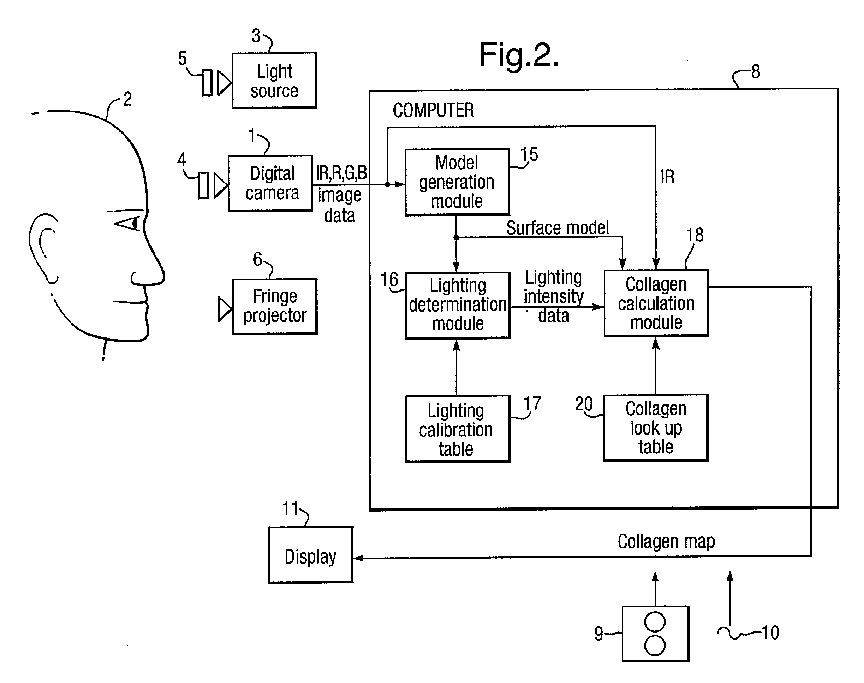

A first system will now be described with reference to Figure 2 which is a schematic block diagram of a collagen thickness measurement system.

In accordance with this system, a digital camera 1 operable to obtain red, green, blue and infra-red images is provided which is arranged to obtain an image of an individual 2 illuminated by a light source 3. A first polarising filter 4 is provided in front of the lens of the digital camera 1 and a second polarising filter 5 cross polarised with the first is provided in front of the light source 3. Also provided is a fringe projector 6 which is arranged to project a regular grid pattern of light in the visible spectrum onto the area of the individual 1 being imaged.

In order to obtain measurements of the concentrations and distribution of chromophores in the papillary dermis 54 and epidermis 52, the effect of reflection of light directly by the cornified layer 50 is required to be removed so that a measurement of the remitted light which has interacted with the chromophores present in the epidermis 52 and papillary dermis 54 can be made. As the interaction of light with collagen 58 in the skin is such to cause the light to lose its polarisation, by providing the cross polarised filters 4, 5, light from the light source 3 passing through the polarising filter 5 in front of the light source 3 which is reflected directly by the cornified layer 50 without interacting with the other layers of the skin is caused to be filtered by the polarising filter 4 in front of the lens of the digital camera 1. The image data obtained by the digital camera 1 is thereby caused to be solely representative of the light remitted by the skin which has interacted with the structures of the epidermis 52 and papillary dermis 54.

The images obtained by the digital camera 1 are then transmitted to a computer 8 which is configured by software either provided on a disk 9 or by receiving an electrical signal 10 by via a communications network to be configured into a number of functional modules 15-20 which cause the computer 4 to process the image data received from the digital camera 1 to generate an output image which is shown on a display 11. As will be described in detail, the processing of the functional modules 15-20 is such to process the image data received from the digital camera 1 and generate a collagen map representative of the thickness of collagen in the area of skin of the individual 2 being imaged.

It will be appreciated that the functional modules 15-20 illustrated in Figure 2 are purely notional in order to assist with the understanding of the working of the claimed invention and may not in certain embodiments directly correspond with blocks of code in the source code for the software. In other embodiments the functions performed by the illustrated functional modules 15-20 may be divided between different modules or may be performed by the re-use of the same modules for different functions.

In this example the functional modules 15-20 comprise: a model generation module 15 for processing RGB image data of an individual 2 on to which the fringe projector 6 projects a regular grid pattern to generate a 3-D wire mesh model of the surface being imaged; a lighting determination module 16 and a lighting calibration table 17 for processing a model generated by the model generation module 15 to determine an estimate of the strength of illumination of the surface of the individual's skin by the light source 3; and a collagen calculation module 18 and a collagen look-up table 20 which together process an infra-red image of the individual, the wire mesh model generated by the model generation module 15 and lighting intensity data received from the lighting determination module 16 to generate a collagen map indicative of the thickness of collagen in the area of the individual's skin being imaged.

It is known that measurements of the thickness of the papillary dermal layer 54 (collagen thickness) can be determined from image values measured by a digital camera 1 when skin is illuminated with light of having a known spectrum. Where the surface geometry of an area of skin is not substantially flat, it is, however, not possible to control lighting conditions to eliminate lighting variation. To overcome this problem, rather than attempting to control the lighting of an area of skin in this example the model generation module 15, lighting determination module 16, lighting calibration table 17 and collagen calculation module 18 co-operate to enable an estimated measurement of light intensity remitted from the surface of the skin independent of the skin's histology to be determined. This measurement therefore identifies variations arising due to variations in illumination and surface geometry. A measurement of collagen thickness at points of the surface of skin can then be determined by normalising received infra-red lighting intensity values to account for the identified variations in illumination and surface geometry and then converting the normalised data into collagen thickness measurements.

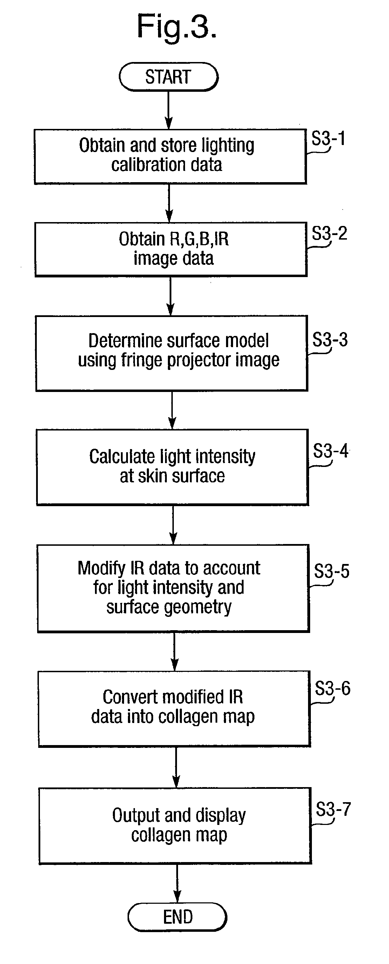

The detailed processing of the collagen thickness measurement apparatus of Figure 2 will now be described in detail with reference to Figure 3 which is a flow diagram of the processing undertaken by the collagen thickness measurement apparatus of Figure 2.

Processing Undertaken by Collagen Thickness Measurement Apparatus

Before any images of an individual are obtained, the apparatus is first calibrated (S3-1) by storing lighting calibration data in the lighting calibration table 17. This lighting calibration data is indicative of the manner in which the infra-red light intensity of light generated by the light source 3 varies within a volume. This is achieved by obtaining a series of pictures using the digital camera 1 of a plain flat sheet held at set positions within the volume where an individual 2 will subsequently be imaged illuminated by the light source 3 via the polarising filter 4.

Thus for example in the case of a volume 30cm x 30cm x 30cm a series of images of a blank white sheet held parallel with the image plane of the digital camera 1 at distances within the volume separated by say, for example, 5cm could be obtained. The infra-red values for these images are then stored within the lighting calibration table 17 together with data indicating the distance at which the sheet was held. In this example the digital camera 1 comprises a digital camera operable to obtain red, green, blue and infra-red images. The images obtained by the camera therefore comprise R, G, B, IR values ranging from 0 to 255 for a larger array of pixels where the R, G, B, IR values are indicative at the extent of light received by a photoreceptor within the camera one for each pixel in an image appears to be red, green, blue and infra-red where a completely cold black pixel has R, G, B, IR values of 0,0,0,0 and a hot bright white pixel has R, G, B, IR values of 255, 255, 255, 255. The data stored in the lighting calibration table 17 will therefore comprise for each pixel in an image obtained by the camera, an infra-red light intensity value for each of the distances of the sheet for which calibration data is obtained, where the infra-red light intensity values correspond to the IR values of the obtained images.

Having stored lighting calibration data in the lighting calibration table 17, images of an individual 2 occupying at least part of the volume for which lighting calibration data has been stored is then obtained (S3-2), whilst the fringe projector 6 projects a regular grid pattern onto the surface being imaged. The appearance of this regular grid as reflected by the surface is such that the distortions of the grid vary due to the relative distance of the surface of the individual's skin at different points in an image.

The R, G and B values for the image are then passed to the model generation module 15 which processes (S3-3) the received colour image to generate a wire mesh model representation of the surface of the individual 2 being imaged. This is achieved by processing the R, G, B values of the image including the projected grid in a conventional way such as that undertaken in a Z snapper available from Vialux GmbH, Reichenhainer, Strasse 88, 09126 Chemnitz, Germany. The 3D wire mesh of the individual 2 generated as a result of the processing the model generation module 15 surface model is then passed to both the lighting determination module 16 and the collagen calculation module 18.

When the lighting determination module 16 receives a surface model, the lighting determination module proceeds (S3-4) to calculate for each pixel in the image obtained by the digital camera 1, the intensity of infra-red light impinging on the surface of the skin of the individual 2 being imaged as perceived by the camera 1 via the polarising filter 4.

This is achieved by the lighting determination module 16 calculating for each pixel in an image obtained by the digital camera 1, the position on the surface of the wire mesh model generated by the model generation module 15 which corresponds to the centre of the pixel. The lighting determination module 16 then interpolates an infra-red lighting intensity value for the pixel from the lighting calibration table 17 values for the two distances closest to the position the pixel represents. In this example, this is achieved through a simple linear interpolation of the infra-red lighting intensity values stored in the lighting calibration table 17 for a pixel based on the relative distance for a point on the surface of the individual 2 relative to the closest positions for which calibration data has been stored.

This process is repeated for each individual pixel in the image obtained by the digital camera 1, and the generated infra-red lighting intensity data representing the intensity with which infra-red light from the light source 3 impinges on the surface of the individual 2 for all of the pixels corresponding to pixels representing the surface of the individual 2 is then passed to the collagen calculation module 18.

When the collagen calculation module 18 receives infra-red lighting intensity data and data representing a surface model, the collagen calculation module 18 proceeds (S3-5) to utilise the surface model data and the infra-red lighting intensity data to determine an expected level of illumination returned by the surface of the individual 2 on the basis of the light source 3 and modify the infra-red channel image received from the digital camera 1 to account for variations arising due to differences in surface geometry and irregular illumination by the light source 3.

More specifically the infra-red lighting intensity data is first processed to modify the data to account for the fact that in contrast to the calibration sheet used to generate the lighting calibration data in the lighting calibration table 17 the surface of an individual to be imaged is not orientated in exactly the same plane as the digital camera 1. The difference in orientation gives rise to two separate effects.

Firstly, the amount of light received by a camera 1 is dependent upon the relative angle of the surface being imaged. In order to account for this variation the lighting intensity data for a particular pixel needs to be modified by a factor proportional to cos θ where θ is the difference between the orientation of the normal of the surface of the individual 2 at the point being imaged relative to a ray of light passing through the centre of the lens of the digital camera normal to the image plane for the digital camera 1.

In addition to a factor proportional to cos θ, in the case of light reflected by skin, the proportion of remitted light is dependent upon internal reflections and interactions with collagen present in the skin. The effect of these internal interactions on the amount of reflected light is proportional to $\cos^2 \theta$.

Thus in this example the lighting intensity data for images obtained by the digital camera are modified by initially identifying for a pixel in a received IR image the point on the surface of the individual 2 corresponding to the pixel. The relative orientation of the surface of the wire mesh model at that point is then compared with a surface parallel to the image plane of the digital camera is then determined. A normalised infra-red measurement for the pixel can then be determined by dividing the infra-red value for a pixel by the corresponding infra-red lighting intensity value multiplied by a correction factor K with:

$$K = Cos\,\theta + \lambda\,Cos^2\,\theta$$

where λ is an experimental value obtained by measuring the reflective properties of skin which can be calculated by taking an average value from a number of different skin samples.

Since the measurement for pixels corresponding to particularly oblique pixels does not return reliable values, the collagen calculation module 18 in this example sets the modified infra-red values for pixels representing oblique surfaces to a null value so that subsequently no collagen map data is generated for such pixels. A typical range for which the collagen calculation module 18 might set values to null values would be for all pixels where θ is greater than 45°.

When this processing has been completed for all of the pixels in the infra-red image, the variations in lighting intensity due to surface geometry and the light source 3 will be removed from the infra-red measurements as they will have been divided by a value representative of the expected level of reflected light for the light source 3 and surface geometry of the individual being imaged in the absence of interactions with any chromophores. These normalised infra-red intensity values are then (S3-6) converted into collagen thicknesses by accessing the collagen look-up table 20 which stores data for the proportion of infra-red light returned by a skin surface for different levels of collagen thickness.

An image illustrating the variation of collagen thickness for the skin of the individual 2 being imaged is then (S3-7) output and displayed on the display screen 11.

Second System

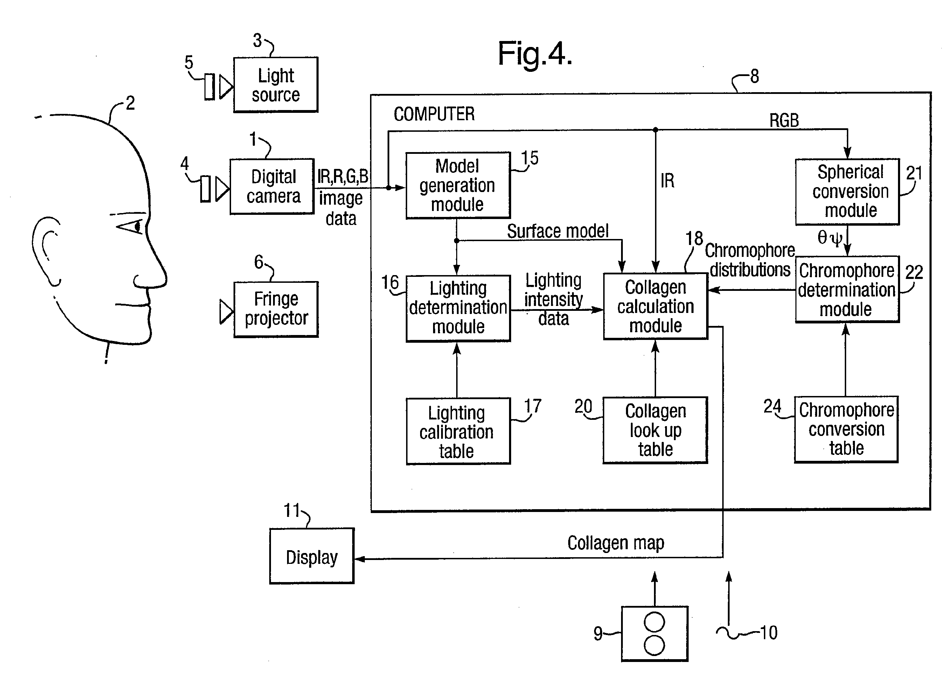

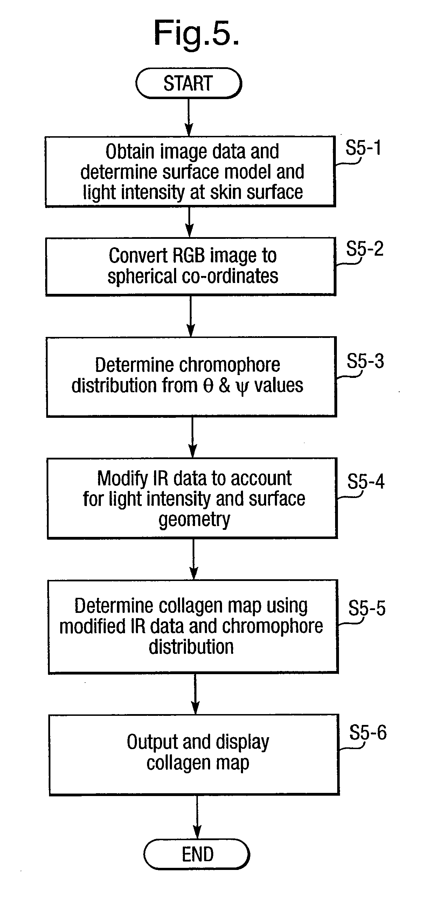

A second system will now be described with reference to Figures 4 and 5.

In the first system a collagen thickness measuring apparatus was described in which collagen thickness is determined on the basis of a series of infra-red image pixel values normalised to account for variations in lighting and surface geometry. Collagen thickness obtained on the basis of infra-red values are usually accurate since light remitted from the surface of the skin in infra-red frequencies is substantially unaffected by the presence of other chromophores such as blood and melanin. It is, however, possible to improve the accuracy of the collagen measurement by accounting for variations arising due to the presence of blood and collagen as will now be described.

Referring to Figure 4 which is a schematic block diagram of a second system, the apparatus of Figure 4 is identical to that of Figure 2 with the exception that three additional functional modules are provided within the memory of the computer 8. These additional modules comprise a spherical conversion module 21, a chromophore determination module 22 and a chromophore conversation table 24. In this example, as will be described, these additional functional modules process the red, green and blue image data obtained by the digital camera 1 to determine the concentrations of blood and melanin present in the skin of an individual being imaged. This chromophore distribution data is then passed to the collagen calculation module 18. The collagen calculation module 18 then utilises this chromophore distribution data together with normalised infra-red pixel data to access a modified collagen lookup table 20 which enables the modified infra-red values and the chromophores distributions to be converted into measurements of collagen thickness.

The processing undertaken by the apparatus of Figure 4 is illustrated in the flow diagram shown in Figure 5.

Initially, as in the previous example, after calibration data has been stored image data is obtained and processed to determine values indicative of infra-red light intensity at the skin surface (S5-1 ). This processing is identical to that described in the previous example in relation to steps (S3-1-S3-4) and will not therefore be repeated here.

In addition to this processing, the RGB values for an image obtained by the digital camera 1 are also passed to the spherical conversion module 21. The spherical conversion module 21 then (S5-2) converts the conventional RGB data for each pixel in an image into a corresponding set of spherical co-ordinates θψr where the spherical angles θ and ψ are substantially indicative of the hue and chromaticity represented by an individual pixel in an image captured by the digital camera 1 and the radial co-ordinate is substantially indicative of the brightness of the pixel.

This conversion is achieved in the conventional manner with:

$$\theta = \cos^{-1}\left( B\left(R^2 + B^2 + G^2\right)^{-\frac{1}{2}} \right)$$

$$\psi = \tan^{-1}\left( \frac{G}{R} \right)$$

and

$$r = \left(R^2 + B^2 + G^2\right)^{\frac{1}{2}}$$

The conversion is performed for each pixel in the original pixel array for the RGB image generated by the digital camera 1. The results of the conversion is a set of spherical $\theta_\psi$ co-ordinates for each pixel in the original image.

The effect of conversion of RGB values into spherical co-ordinates is similar to calculating ratios of colour values in that the obtained θ and $_\psi$ values are independent of lighting intensity and instead are solely dependent upon the concentration of blood and melanin in the skin being imaged. After the spherical conversion module 21 has converted the RGB values for an image into spherical co-ordinates, the array of pairs of the θ and $_\psi$ values is passed to the chromophores determination module 22 which proceeds to process (S5-3) the array to obtain values indicative of the concentration of blood and melanin at individual points on the surface of the skin of the individual 2 being imaged.

In this example this is achieved by processing each pair of θ and ψ values for each pixel in an array in turn by scaling the θ and ψ values so that instead of comprising values between $\pi$ and $-\pi$ and 0 and $\pi/_2$. The scaled θ and ψ values comprise integers of values ranging between 0 and 255. These scaled θ and ψ values are then utilised to access the chromophore conversion table 24 which in this example is a 255 x 255 lookup table associating pairs of scaled θ and ψ value co-ordinates with pairs of concentrations of blood and melanin liable to give rise to such scaled θ and ψ values. In this example the chromophore conversion table 24 comprises a table associating blood and melanin concentrations with various θ and ψ values, where the θ and ψ values fall within the expected range of the colour space for skin. In the event that the combination of θ and ψ values for a particular pixel falls outside the range for which chromophore concentration data is stored within the chromophore conversion table 24, in this example the chromophore determination module 22 returns a null value for the concentration of blood and melanin for the pixel with θ and ψ values for the pixel. This chromophore data is then passed to the collagen calculation module 18.

At this stage the collagen calculation module 18 will be in receipt of a surface model representing surface of the individual 2 being imaged, infra-red lighting intensity data representing the infra-red light intensity impinging on the surface of the individual 2 which would be received by the digital camera 1 if the skin surface shared the properties of the calibration sheet for which lighting calibration data 17 is stored in the memory of the computer 8, an infra-red image and a pair of blood and melanin concentration values for each pixel in an image. The collagen calculation module 18 then, as in the previous example, proceeds (S5-4) to determine an angle for the relative orientation of the surface of the skin of the individual 2 for each pixel in the image, and to normalise the received infra-red data to account for variations due to surface geometry, using the lighting intensity data and the 3D surface model in the same way as was previously described in relation to the first example.

The collagen calculation module 18 then (S5-5) calculates collagen thickness values for each pixel in the image using the modified infra-red value and the chromophore distribution data by accessing a modified collagen lookup table 20 where the modified collagen lookup table 20 stores a collagen thickness measurement values for all possible combinations of blood and melanin concentrations and modified infra-red values which are expected for skin samples.

When this has been repeated for each of the individual pixels in the infra-red image and its associated chromophore distribution values and a collagen map based on the obtained collagen thickness value is output and displayed (S5-6).

Thus in this way by determining an array of blood and melanin concentrations and storing data in the collagen lookup table 20 associating infra-red values and chromophore concentrations with collagen thickness an improved collagen thickness measure can be obtained which accounts for variations in returned levels of infra-red light which arise due to the presence of blood and melanin.

Third System

A Third system will now be described. In the previous two systems the expected level of light remitted from the surface of an individual's skin is calculated by generating a 3D wire mesh model of the surface of an area of skin and accessing stored lighting intensity data. In this embodiment an alternative system will be described in which lighting intensity data is determined directly from image data received by a digital camera 1 in the absence of any fringe projection system 6.

As with the first two example the apparatus in this example comprises a digital camera 1 arranged to obtain an image of an individual 2 illuminated by a light source 3 via polarising filter 5. Again as in the first two embodiments another polarising filter 4 is provided in front of the lens of the digital camera 1 arranged so as to be cross polarised with the polarising filter 5 in front of the light source 3.

In this example the digital camera 1 is arranged to obtain red, green, blue and infra-red images which are passed to a computer 8 which processes the data and generates a collagen map representative of collagen thickness which is shown on a display 11. To that end the computer 8 is configured either by a disc 9 or an electrical signal 10 into a number of functional modules 18-34, which in this example comprise: a collagen calculation module 18, a collagen lookup table 20, a spherical conversion module 21, a chromophore determination module 22 and a chromophore conversion table 24 similar to those previously described in relation to the second example. The functional modules in this example also additionally comprise: an image generation module 30, an inverse conversion table 32 and an illumination determination module 34.

As will be described in detail later, the processing conducted by the image generation module 30 and the inverse conversion table 32 is such to process a determined chromophore distribution generated by the chromophore determination module 20 to generate a derived image representative of the appearance of determined blood and melanin concentrations under uniform lighting conditions in the absence of any variation in collagen thickness. This derived image is then compared with the RGB image received from a digital camera 1 by the illumination determination module 34 which calculates illumination intensity data indicative of lighting intensity variations. This lighting intensity data is then utilised by the collagen calculation module 18 to normalise infra-red image data to eliminate variations arising due to lighting variations. This normalised infra-red data is then utilised together with the chromophore distribution data determined by the chromophore determination module 22 to access collagen thickness measurements stored within the collagen lookup table 20 in a similar way to the processing undertaken by the collagen calculation module 18 in the second example.

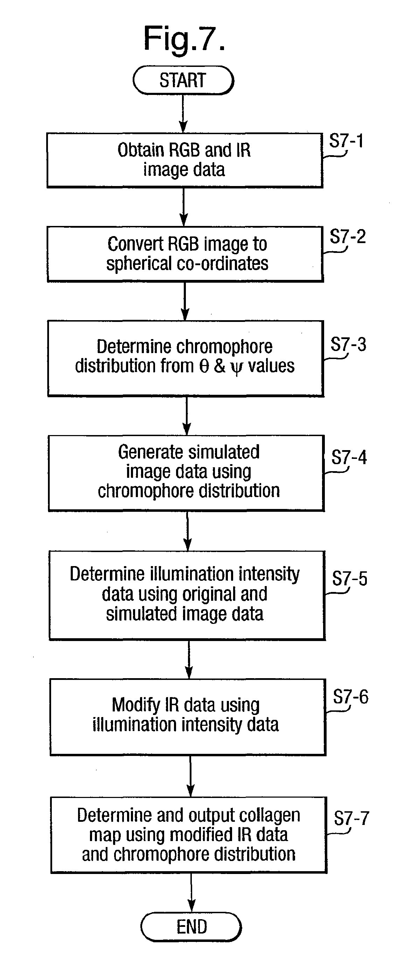

Figure 7 is a flow diagram of the processing of the computer 8 undertaken in this example of the invention. Initially (S7-1) the digital camera 1 obtains RGB and infra-red image data of the individual 2 is illuminated by the light source 3. The RGB portion of the image data is then passed to the spherical conversion module 21 which converts the RGB data into spherical $\theta\psi r$ co-ordinates (S7-2) in exactly the same way as has been previously described in relation to step (S5-2) of the second example.

The $\theta$ and $\psi$ values determined by the spherical conversion module are then passed to the chromophore determination module 22 which converts the $\theta$ and $\psi$ values into blood and melanin concentration values by accessing the chromophore conversion table 24 in exactly the same way as previously described in relation to step (S5-3) of the second example.

This chromophore distribution data is then passed together with the r luminosity values generated by the spherical conversion module 21 to the image generation module 30 which generates (S7-4) a simulated image using this data. More specifically, the image generation module 30 proceeds to utilise the chromophore distribution data to access an inverse conversion table 32 which is a lookup table associating blood and melanin concentrations with corresponding θ and ψ values representing the hue and chromaticity of skin containing the identified chromophore concentrations under uniform lighting conditions and with uniform collagen thickness. This inverse conversion table 32 is therefore data representative of an inverse function corresponding to the function for converting θ and ψ values to measurements of blood and melanin stored in the chromophore conversion table 24. In the case of pixels which are associated with null values within the chromophore distribution, no θ and ψ values are determined.

The image generation module 30 then generates a derived image based on the determined levels of blood and melanin concentrations by converting the generated θ and ψ values and the r values received from the original spherical conversion module 21 into RGB data using the following equations:

$$R = r\,sin\theta\;cos\psi$$

$$G = r\,sin\theta\;sin\psi$$

$$B = r\,cos\theta$$

This derived image is then passed to the illumination determination module 34.

The illumination determination module 34, at this point, determines (S7-5) illumination intensity data by calculating the difference in R values for corresponding pixels in the original and derived images. Since the image data generated by the image generation module 30 is generated using an inverse conversion table 32 which represents the expected appearance of a pixel having the identified chromophore distributions under uniform lighting conditions, the effect of this differencing operation is to obtain an indication of the manner in which the lighting of the individual 2 by the light source 3 is non-uniform due to variations in light intensity and surface geometry.

This illumination intensity data is then passed to the collagen calculation module 18 which modifies (S7-6) the infra-red pixel data by dividing each of the infra-red pixel data by the illumination intensity data for the red channel for that pixel in the image.

The collagen calculation module 18 then (S7-7) calculates for each pixel a collagen thickness measurement by utilising the modified infra-red data and the chromophore distribution data for blood and melanin associated with each pixel to lookup corresponding collagen thickness measurement in the collagen lookup table 20 in the same way as has previously been described in relation to the second example and a generated collagen map is then output and displayed on the display 11.

Alternative Embodiments and Modifications

In the first example normalised lighting intensity values are stated to be calculated by using a correction factor dependent upon A, an experimental value obtained by measuring respective properties of the skin calculated by taking an average value from a number of different skin samples. The proportion of light reflected by the skin is dependent upon qualities of collagen which vary with age. In one example a series of λ values could be stored for different ages and the age of an individual being imaged could be utilised to select an appropriate A value. Alternatively a λ value could be calculated specifically for an individual by taking a sample of imaged skin for a portion of an individual where the collagen thickness is known to be substantially constant. A suitable area to be sampled would be an area across the forehead of the face. The extent to which infra-red light is remitted from such a sample area could be utilised to select an appropriate A value for normalising light intensities.

In the above described embodiments collagen thickness values have been described as being calculated based on a measurement of the remittance of infra-red light by skin. As stated previously measurements using infra-red light are preferable since the remittance of infra-red light is substantially unaffected by the presence of other chromophores. In other embodiments other wavebands could be utilised to obtain measurements of collagen thickness. The use of other wavebands would, however, only be possible if concentrations of other chromophores effecting measurements could be obtained such as is described in the second and third embodiments.

In the third example light intensity variation data is stated as being derived from differences in red channel data, it would be appreciated that other methods could be used to determine the returned light intensity. Thus for example other colour channels could be utilised. Alternatively an average intensity of a number of different colour channels might be utilised instead.

Although in the second and third example systems have been described in which blood and melanin concentration data is determined utilising a lookup table associating spherical co-ordinates with chromophore concentrations, it will be appreciated that alternative means exist for measuring approximations of chromophore concentration. Thus for example instead of undertaking a transformation to spherical co-ordinates, ratios of colour values could be utilised. A further alternative would be to undertake a principal component analysis of the variations appearing in an image since variations in blood and melanin correlate reasonable well with the principal variations in apparent colour for a sample area of skin.

In the first two embodiments 3D model data is described as being obtained using a fringe projector 6 and processing obtained image data in a similar way to the processing of data using a Z snapper such as available from Vialux GmbH. It would be appreciated that any suitable method of generating a 3D dimensional wire mesh model of the surface of an individual 2 being imaged could be used instead. Thus for example instead of having a system based on the projection of the regular grid onto a surface a stereoscopic imaging system could be used to generate a model surface.

Although the embodiments of the invention described with reference to the drawings comprise computer apparatus and processes performed in computer apparatus, the invention also extends to computer programs, particularly computer programs on or in a carrier, adapted for putting the invention into practice. The program may be in the form of source or object code or in any other form suitable for use in the implementation of the processes according to the invention. The carrier can be any entity or device capable of carrying the program.

For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal which may be conveyed via electrical or optical cable or by radio or other means.

When a program is embodied in a signal which may be conveyed directly by a cable or other device or means, the carrier may be constituted by such cable or other device or means.

Alternatively, the carrier may be an integrated circuit in which the program is embedded, the integrated circuit being adapted for performing, or for use in the performance of, the relevant processes.

**Claims**

1.    Apparatus for measuring skin collagen thickness comprising:

a camera (1) operable to obtain an image of an area of illuminated skin (2);
an illumination determination module comprising:

a lighting calibration table (17) storing data indicative of measurements of the manner in which lighting intensity varies in a volume where a sample of skin (2) is to be imaged;
a model generation module (15) operable to process a received image to generate a 3 dimensional model of the surface of the area of skin (2) being imaged; and
a surface lighting determination module (16) operable to process a 3 dimensional model generated by the model generation module and the data stored in the lighting calibration table (17) to determine an estimate of the manner in which light returned by an area of skin (2) being imaged varies due to variations in lighting intensity and surface geometry;

an illumination normalisation module (18) operable to process at least one colour channel of an obtained image on the basis of the determined variations; and
a collagen determination module (20) operable to determine a measurement of collagen thickness utilising a processed colour channel of an obtained image.

2.    The apparatus of claim 1 further comprising a projector (6) operable to project a pattern onto the surface of an area of skin (2) being imaged, wherein said model generation module (15) is operable to process a received image of an area of skin (2) onto which a pattern is projected by said projector (6) to generate a 3 dimensional model of the surface of the area of skin (2) being imaged.

3.    The apparatus of claim 1 or claim 2 in which the lighting determination module is configured to (S3-4) to calculate for each pixel in an image obtained by the camera (1) the position on the surface of the model generated by the model generation module (15) which corresponds to the centre of the pixel and interpolate a lighting intensity value for the pixel from the lighting calibration table (17) values for the two distances closest to the position the pixel represents.

4. The apparatus of claim 3 in which the lighting determination module is configured to repeat the calculation and interpolation steps for each individual pixel in the image obtained by the camera (1), and pass to the collagen calculation module (18) the generated infra-red lighting intensity data representing the intensity with which infra-red light from the light source (3) impinges on the surface of the skin for all of the pixels.

5. The apparatus of any preceding claim wherein the surface lighting determination module (16) is operable to process a 3 dimensional model generated by the model generation module and the data stored in the lighting calibration table (17) to determine an estimate of an expected intensity of light returned by an area of skin (2) being imaged by:

   determining an estimate of the intensity of light impinging on the surface of skin (2) being imaged; and determine an expected intensity of light returned by an area of skin (2) by modifying the estimated intensity of light impinging on the surface of skin (2) on the basis of the relative orientation of the surface of the skin relative to an image plane of a camera (1) obtaining an image.

6. The apparatus of any preceding claim wherein said collagen determination module (20) comprises a look up table associating measurements of a processed colour channel with thickness of collagen with which return said measurements of a processed colour channel.

7. The apparatus of any of claims 1-5 further comprising:

   a chromophore measurement module (21,22,24) operable to process a received image to determine an estimate of the concentrations and distribution of blood and melanin in an area of skin (2) appearing in an obtained image; wherein the collagen determination module (20) is operable to determine a measurement of collagen thickness utilising estimates of the concentrations and distribution of blood and melanin in an area of skin (2) generated by said chromophore measurement module (21,22,24) and the processed colour channel of an obtained image.

8. The apparatus of claim 7 wherein said collagen determination module (20) comprises a look up table associating measurements of a processed colour channel and concentrations of blood and melanin with thickness of collagen and concentrations of blood an melanin with which return said measurements of a processed colour channel.

9. The apparatus of any preceding claim further comprising:

   a light source (3,5) operable to illuminate an area of skin (2) with polarised light; wherein said camera (1) is operable to obtain an image of an area of skin (2) illuminated by said light source (3,5) via a polarising filter (4) operable to filter light having the same polarization with which said light source (3,5) is operable to illuminate said area of skin (2).

10. A method of measuring skin collagen thickness comprising:

   obtaining (s3-2) an image of an area of illuminated skin (2);
   storing (s3-1) measurements of the manner in which lighting intensity varies in a volume where a sample of skin (2) is to be imaged;
   processing (s3-3) an obtained image to generate a 3 dimensional model of the surface of the area of skin (2) being imaged;
   utilising (s3-4) the generated 3 dimensional model and the stored lighting measurements to determine an estimate of the manner in which light returned by an area of skin (2) being imaged varies due to variations in lighting intensity and surface geometry;
   processing (s3-5) at least one colour channel of the obtained image on the basis of the determined variations; and determining (s3-6) a measurement of collagen thickness utilising the processed one colour channel of the obtained image.

11. The apparatus of claim 10 further comprising projecting a pattern onto the surface of an area of skin (2) being imaged and processing a received image of an area of skin (2) onto which a pattern is projected to generate a 3 dimensional model of the surface of the area of skin (2) being imaged.

12. The apparatus of claim 10 or 11 further comprising calculating for each pixel in an image obtained by the camera (1) the position on the surface of the model generated by the model generation module (15) which corresponds to the centre of the pixel and interpolating a lighting intensity value for the pixel from the lighting calibration table (17)

values for the two distances closest to the position the pixel represents.

13. The method of claim 10, 11 or 12 wherein utilising (s3-4) a generated 3 dimensional model generated to determine an estimate of an expected intensity of light returned by an area of skin (2) being imaged comprises:

determining an estimate of the intensity of light impinging on the surface of skin (2) being imaged; and determining an expected intensity of light returned by an area of skin (2) by modifying the estimated intensity of light impinging on the surface of skin (2) on the basis of the relative orientation of the surface of the skin relative to an image plane of a camera (1) utilising (s3-4) the generated 3 dimensional model of the surface of the skin being imaged.

14. The method of claims 8 or claim 9, further comprising:

processing (s5-1-s5-3) a received image to determine an estimate of the concentrations and distribution of blood and melanin in an area of skin (2) appearing in an obtained image; wherein determining (s5-5) a measurement of collagen thickness comprises determining a measurement of collagen thickness utilising the determined estimates of the concentrations and distribution of blood and melanin in an area of skin (2).

15. A computer readable medium (9,10) comprising computer interpretable instructions which when executed by a programmable computer (8) cause the computer to perform a method in accordance with any of claims 10-14.

# Fig.1.

Incident light

Remitted light

57

58

57

58

58

55

58

58

EP 2 340 763 A1

Fig.2.

# Fig.3.

START

Obtain and store lighting calibration data — S3-1

Obtain R,G,B,IR image data — S3-2

Determine surface model using fringe projector image — S3-3

Calculate light intensity at skin surface — S3-4

Modify IR data to account for light intensity and surface geometry — S3-5

Convert modified IR data into collagen map — S3-6

Output and display collagen map — S3-7

END

Fig.4.

3

5

Light source

4

1

Digital camera

IR,R,G,B image data

2

6

Fringe projector

8

COMPUTER

RGB

Model generation module 15

IR

Spherical conversion module 21

Surface model

θ ψ

18 Chromophore distributions

16 Lighting determination module

Lighting intensity data

Collagen calculation module

Chromophore determination module 22

Lighting calibration table 17

20 Collagen look up table

24 Chromophore conversion table

11

Display

Collagen map

9

10

# Fig.5.

START

Obtain image data and
determine surface model and
light intensity at skin surface — S5-1

Convert RGB image to
spherical co-ordinates — S5-2

Determine chromophore
distribution from $\theta$ & $\psi$ values — S5-3

Modify IR data to account
for light intensity and surface
geometry — S5-4

Determine collagen map using
modified IR data and chromophore
distribution — S5-5

Output and display
collagen map — S5-6

END

# Fig.6.

# Fig.7.

```
          ( START )
              |
              v
  +------------------------+
  |   Obtain RGB and IR    |  S7-1
  |      image data        |
  +------------------------+
              |
              v
  +------------------------+
  |  Convert RGB image to  |  S7-2
  |  spherical co-ordinates|
  +------------------------+
              |
              v
  +------------------------+
  |  Determine chromophore |  S7-3
  | distribution from θ & ψ values |
  +------------------------+
              |
              v
  +------------------------+
  |   Generate simulated   |  S7-4
  |    image data using    |
  | chromophore distribution|
  +------------------------+
              |
              v
  +------------------------+
  | Determine illumination intensity |  S7-5
  |   data using original and   |
  |    simulated image data     |
  +------------------------+
              |
              v
  +------------------------+
  |    Modify IR data using    |  S7-6
  |  illumination intensity data |
  +------------------------+
              |
              v
  +------------------------+
  | Determine and output collagen |  S7-7
  |   map using modified IR data   |
  |   and chromophore distribution  |
  +------------------------+
              |
              v
           ( END )
```

Europäisches
Patentamt

European
Patent Office

Office européen
des brevets

# EUROPEAN SEARCH REPORT

Application Number

EP 11 15 4239

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2005/251049 A1 (CANE MICHAEL R [GB] ET AL CANE MICHAEL ROGER [GB] ET AL) 10 November 2005 (2005-11-10) * abstract * * paragraph [0010] - paragraph [0040] * * paragraph [0048] - paragraph [0077] * * figures 1,2 * | 1-15 | INV. A61B5/00 A61B5/103 |
| A | US 6 324 417 B1 (COTTON SYMON D OYLY [GB]) 27 November 2001 (2001-11-27) * abstract * * column 1, line 51 - column 3, line 10 * * column 6, line 27 - column 7, line 5 * * figures 1,2 * * claims 1-21 * | 1-15 | |
| A | WO 97/47942 A (LEUVEN K U RES & DEV [BE]; PROESMANS MARC RAPHAEL ANTOON [BE]; GOOL LU) 18 December 1997 (1997-12-18) * abstract * * page 2, line 6 - page 9, line 7 * * figures 8-10 * | 1-15 | |
| A | GB 2 361 994 A (ASTRON CLINICA LTD [GB]) 7 November 2001 (2001-11-07) * abstract * * page 4, line 1 - page 5, line 19 * * page 11, line 25 * * figure 7 * | 1-15 | TECHNICAL FIELDS SEARCHED (IPC) A61B |
| A | WO 01/50948 A2 (INSTRUMENTATION METRICS INC [US]) 19 July 2001 (2001-07-19) * page 6, line 29 - page 10, line 17 * * figure 1 * | 1-15 | |

-/--

The present search report has been drawn up for all claims

| Place of search | Date of completion of the search | Examiner |
|---|---|---|
| Munich | 27 May 2011 | Doyle, Aidan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
...........................................................................................
& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 11 15 4239

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GB 2 429 523 A (ASTRON CLINICA LTD [GB]) 28 February 2007 (2007-02-28) * abstract * * page 4, line 15 - page 5, line 10 * * page 8, line 19 - page 11, line 2 * * page 12, line 5 - page 14, line 23 * * page 18, line 19 - page 30, line 11 * * figures 1,3 * | 1-15 | |

TECHNICAL FIELDS
SEARCHED      (IPC)

The present search report has been drawn up for all claims

| Place of search | Date of completion of the search | Examiner |
|---|---|---|
| Munich | 27 May 2011 | Doyle, Aidan |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.......................................................................................

& : member of the same patent family, corresponding
document

EPO FORM 1503 03.82 (P04C01)

EP 2 340 763 A1

## ANNEX TO THE EUROPEAN SEARCH REPORT
## ON EUROPEAN PATENT APPLICATION NO.

EP 11 15 4239

This annex lists the patent family members relating to the patent documents cited in the above-mentioned European search report.
The members are as contained in the European Patent Office EDP file on
The European Patent Office is in no way liable for these particulars which are merely given for the purpose of information.

27-05-2011

| Patent document cited in search report | | Publication date | Patent family member(s) | | Publication date |
|---|---|---|---|---|---|
| US 2005251049 | A1 | 10-11-2005 | NONE | | |
| US 6324417 | B1 | 27-11-2001 | AT | 217172 T | 15-05-2002 |
| | | | AU | 725766 B2 | 19-10-2000 |
| | | | AU | 4961597 A | 10-06-1998 |
| | | | BR | 9713097 A | 28-03-2000 |
| | | | CA | 2272290 A1 | 28-05-1998 |
| | | | DE | 69712533 D1 | 13-06-2002 |
| | | | DE | 69712533 T2 | 29-08-2002 |
| | | | EP | 1006876 A1 | 14-06-2000 |
| | | | ES | 2175373 T3 | 16-11-2002 |
| | | | WO | 9822023 A1 | 28-05-1998 |
| | | | GB | 2334099 A | 11-08-1999 |
| | | | IL | 130008 A | 23-11-2003 |
| | | | JP | 2001504020 T | 27-03-2001 |
| WO 9747942 | A | 18-12-1997 | AT | 253721 T | 15-11-2003 |
| | | | AU | 712090 B2 | 28-10-1999 |
| | | | AU | 3085297 A | 07-01-1998 |
| | | | BR | 9709679 A | 11-01-2000 |
| | | | CA | 2256587 A1 | 18-12-1997 |
| | | | DE | 69726025 D1 | 11-12-2003 |
| | | | DE | 69726025 T2 | 16-09-2004 |
| | | | EP | 1009969 A1 | 21-06-2000 |
| | | | IL | 127534 A | 26-08-2001 |
| | | | JP | 2000512012 T | 12-09-2000 |
| | | | KR | 20000016663 A | 25-03-2000 |
| | | | NO | 985746 A | 12-02-1999 |
| | | | US | 2002057832 A1 | 16-05-2002 |
| GB 2361994 | A | 07-11-2001 | EP | 1282382 A1 | 12-02-2003 |
| | | | WO | 0185028 A1 | 15-11-2001 |
| | | | US | 2003139672 A1 | 24-07-2003 |
| WO 0150948 | A2 | 19-07-2001 | AU | 3278901 A | 24-07-2001 |
| | | | US | 2003040664 A1 | 27-02-2003 |
| | | | US | 2001041829 A1 | 15-11-2001 |
| GB 2429523 | A | 28-02-2007 | AU | 2006252181 A1 | 12-07-2007 |
| | | | CA | 2572061 A1 | 23-06-2007 |
| | | | EP | 1810614 A2 | 25-07-2007 |
| | | | US | 2007161910 A1 | 12-07-2007 |

EPO FORM P0459

For more details about this annex : see Official Journal of the European Patent Office, No. 12/82

24

## REFERENCES CITED IN THE DESCRIPTION

**Patent documents cited in the description**

- WO 04010862 A